# EUROPEAN PATENT SPECIFICATION

(11) **EP 0 904 786 B1**
(45) Date of publication and mention of the grant of the patent: **15.12.2004**
(21) Application number: 97114514.9
(22) Date of filing: 22.08.1997
(51) Int. Cl.: A61K 39/00, A61K 48/00

(54) **Tumor vaccination by use of autologous or HLA-related antigen presenting cell (APC) transduced with a tumour antigen and a foreign antigen capable of causing an immune reaction**
Tumorvakzinierung mittels Verwendung autologer oder HLA-verwandter Antigen präsentierender Zellen (APC), die mit einem Tumorantigen sowie einem eine Immunantwort hervorrufenden Fremdantigen transduziert sind
Vaccination anti-tumorale utilisant des cellules présentant l'antigène (CPA) autologues ou apparentées au système HLA, transduites avec un antigène tumoral et avec un antigène étranger capable de causer une réaction immunitiaire

(43) Date of publication of application: 31.03.1999
(73) Proprietor: Science Park Raf S.p.A., 20132 Milano (IT)
(72) Inventor: Bordignon, Claudio, 20132 Milano (IT); Traversari, Catia, 20132 Milano (IT)
(74) Representative: Mallalieu, Catherine Louise

(56) References cited:
- WO-A-89/07946
- WO-A-92/05262
- WO-A-95/06723
- WO-A-97/19169
- FEARON ET AL: "INDUCTION IN A MURINE TUMOR OF IMMUNOGENIC VARIANTS BY TRANSFECTION WITH A FOREIGN GENE" CANCER RESEARCH, vol. 48, 1988, pages 2975-2980, XP002056093
- VILE ET AL: "GENERATION OF AN ANTI-TUMOUR IMMUNE RESPONSE IN A NON-IMMUNOGENIC TUMOUR: HSVTK KILLING IN VIVO STIMULATES A MONONUCLEAR CELL INFILTRATE AND A TH1-LIKE PROFILE OF INTRATUMOURAL CYTOKINE EXPRESSION" INTERNATIONAL JOURNAL OF CANCER, vol. 71, 10 April 1997, pages 267-274, XP002056094

## Description

The invention relates to a method of tumor vaccination using autologous or HLA-related antigen presenting cells which are transduced by a tumour antigen and a gene foreign to the patient.

The number of clinical trials based on the infusion of genetically modified cells for both genetic diseases and cancer is increasing at an impressive rate. Independent of the gene transfer tool, the development of immune response against the transgene(s) and the genetically modified cells is a common potential limitation often overlooked, and is undesirable in connection with known methods of gene therapy.

Gene transfer into human cells is a potential new treatment modality for a variety of genetic and acquired human diseases (Anderson, W.F., 1992; Miller, A.D., 1992). Genetic diseases, due to single-gene defect, have been originally proposed as the primary targets of gene therapy. However, the majority of the human gene therapy trials approved so far involves treatment of acquired diseases, such as cancer and AIDS (Rosenberg, S.A., 1992; Vile, R., and Russell, S.J., 1994). One approach to tumor gene therapy relies on the stimulation of the host immune system against tumor antigens by vaccination of the patient with genetically modified tumor cells, in which the transgene (cytokine, allo-HLA, etc.) is aimed at boosting cellular immune response against the tumor cells (Pardol, M.D., 1993; Colombo, MP.aF.G., 1994; Gansbacher, B., 1994). An alternative approach is based on the adoptive transfer of ex vivo generated tumor-specific effectors. The infused effectors are usually autologous or HLA-compatible lymphocytes, in vitro stimulated to proliferate by specific (Rosenberg, S.A., et al., 1988: Helslop, H.E., et al., 1996) as well as unspecific stimuli (Rosenberg, S., Immunol. Today, 1988). Amplification of the effector function of the infused cells has been achieved by their transduction with cytokine-encoding genes (Rosenberg, S.A., et al., 1991; Riddell, S.R., et al., 1996). An extension of this approach is the use of donor lymphocytes in the context of allogeneic bone marrow transplantation (allo-BMT) for leukemia. This is an established strategy (Kolb, H.J., et al., 1990; Cullis, J.O., et al., 1992; Van Rhee, F., et al., 1994) that carries an inherent risk of graft-vs-host disease (GVHD).

A limit of all these strategies could be the selective elimination of the transduced cells by the host immune system. This phenomenon has been described and characterized in trials involving the use of adenoviral vectors (Yang, Y., et al., 1995) in which the immune response is directed against virus-encoded proteins.

Riddell et al. (1996) report an elimination of genetically modified cells in HIV patients which was related to their immune unbalance.

According to WO 95/06723, BMT-donor lymphocytes (lymphocytes of a donor from whom bone marrow transplants were used for the same patient) have been genetically modified to prevent or treat leukemic relapse and other complications related to severe immunosuppression. BMT-donor lymphocytes were engineered to express a cell surface marker, e.g. the truncated form of the human low affinity nerve growth factor receptor (LNGF-R), for the selection of transduced cells and their in vivo follow up and the HSV-Tk/neo (TN) fusion protein, a bifunctional protein conferring both the neomycin resistance and the HSV-Tk activity for the in vivo ganciclovir-mediated elimination of the transduced cells. Ganciclovir was utilized to control graft-vs-host disease associated to this treatment. A specific response against cells expressing the TNK fusion protein was observed in a patient leading to complete elimination of all transduced cells and to strong resistance to further treatment. Ex vivo analysis of the specificity of such immune response confirmed a progressive increase in the frequency of TN-specific effectors, but could not detect any response against the cell surface marker LNGF-R

It is known from Vile, RG., et al., International Journal of Cancer 71 (1997) 267-274 that in a non-immunogeneic tumor an antitumor immune response can be generated. It was shown that HSV-tk killing in vivo stimulates the mononuclear cell infiltrate and the Th1-like profile of intratumoural cytokine expression. The authors propose a model for the induction of antitumor immunity using suicide genes and discuss the development of improved vectors for gene therapy to augment these effects in vivo. However, this effect is only observed upon exposure to ganciclovir.

Vile, RG., et al., in Int. J. Cancer 71 (1997) 267-274, describe the generation of an antitumor immune response in a nonimmunogeneic tumor. They further describe that HSV-tk killing in vivo stimulates a mononuclear cell infiltrate and a Th1-like profile of intratumoral cytokine expression. It is shown that Tk⁺ tumors being killed in vivo in the presence of ganciclovir induce a pronounced intratumoral infiltrate consisting of macrophages as well as CD4⁺ and CD8⁺ T cells. However, this immunogeneic response is caused by mechanisms which lead to cell death, predominantly by necrosis and exposure to ganciclovir.

There is still a need for a simple and effective method of tumor vaccination. The invention provides such a method.

### Summary of the Invention

In one aspect the invention comprises use of an autologous or HLA-related antigen presenting cell (APC) which is capable of presenting an antigen in a patient, said cell being transduced with a tumor antigen and a foreign antigen capable of causing an immune reaction in a patient's body, for the preparation of a medicament for at least two subsequent administrations for tumor vaccination.

The invention further comprises a use for tumor vaccination wherein the cells preferably are transduced with a bacterial gene or an animal gene.

Such a bacterial gene preferably is an antibiotic resistance gene, such as the neo gene, or a HSV gene, e.g., the HSV-tk gene. Preferably, the target cells are transduced in vitro.

The invention further comprises said use for tumor vaccination for the treatment of neoblastoma and brain tumors.

It is further preferred to administer to the patient in addition a cytokine to cause local inflammation at the site where tumor vaccination occurs. The transfection which causes vaccination can be carried out locally, systemically, or as an ex vivo application of the tumor cells. In the ex vivo application, the transduced target cells are administered to the patient suffering from a tumorous disease.

In another aspect the invention comprises a method for preparing an APC according to the invention comprising transducing ex vivo the APC with a tumor antigen and a foreign antigen capable of causing an immune reaction in a patient's body.

In another aspect the invention comprises an antigen presenting cell transduced with a tumor antigen and a foreign antigen capable of causing an immune reaction in a patient's body.

### Detailed description of the invention

It has surprisingly been found that an effective tumor vaccination can be achieved by repeatedly applying to a patient human cells which are capable of presenting antigens and which in addition contain a foreign antigen capable of being expressed and presented in these cells. It has surprisingly turned out that a lytic activity of the patient's responder cells against the stimulator cells occurs already after at least one repeated immunostimulation with such cells. Suitably, however, the infusion with stimulator cells is performed several times at increasing doses. Preferred are 4 or 10 infusions, particularly preferred are 8 to 10 infusions. The total dose is preferably in the range between 10⁶ cells/kg and 10¹¹ cells/kg. It has turned out that it is advantageous to administer a total dose between 10⁸ and 10¹¹ transduced cells/kg. The single dose is preferably in the range between 10⁵ cells/kg and 10⁸ cells/kg. Autologous or HLA-identical antigen presenting cells are used.

It was known from the state of the art that administration of genetically modified cells to a patient might cause immune reactions. So far, however, such immune reactions had been only an undesirable side-effect (GvHD). This effect can be prevented by using a suicide gene in combination with a pro-drug (HSVtk/GC killing system).

"Autologous cells" in the sense of the present invention are patients' cells which are capable of presenting antigens, in particular tumor antigens, on the cell surface. Such cells are, for instance, tumor cells or antigen presenting cells, such as T cells, macrophages, neutrophils monocytes, and dendritic cells.

"HLA-related cells" in the sense of the present invention means that these cells should be HLA-matched with the recipient's (patient's) cells. Suitably, HLA matching should be such that the bone marrow cells from donors are suitable for BMT. This means that there is to a large extent HLA correspondence, preferably HLA identity, between the donor and the recipient. HLA types and HLA typing are described, for instance, in Williams Hematology, eds. E. Beutler et al., 5th edition (1979) McGraw-Hill, pp. 1611-1617. It is especially preferred that HLA I-related antigens match to a large extent or are identical.

"Presenting an antigen" in the sense of the present invention means that such a cell is capable of presenting an antigen in an MHC Class 1 or, preferably, Class 2 complex on its surface. Such complexes are recognized then by helper T cells or cytotoxic T cells, for instance.

Tumor antigens in the sense of invention are antigens such as for instance MAGE, BAGE and GAGE that are shared between melanomas and other tumors of various histological type but not by normal tissues other than testis and placenta (Van der Bruggen et al., Science 254 (1991) 1643-1647; Boel et al., Immunity 2 (1995) 167-175; Van den Eynde et al., J. Exp. Med 182 (1995) 689-698) or tissue-specific tumor antigens such as for instance CTL recognizing epitopes from tyrosinase (Brichard et al., J. Exp. Med. 178 (1993) 489-49513), MelanA ^{Mart1} (Coulie et al., J. Exp. Med. 180 (1994) 35-42; Castelli et al., J. Exp. Med 181 (1995) 363-368; Kawakami et al., Proc. Natl. Acad. Sci. USA 91(1991) 3515-3519), gp100 ^{mel7} (Bakker et al., J. Exp. Med. 179 (1994) 1005-1009; Kawakami et al., Proc. Natl. Acad Sci. USA 91 (1994) 6458-6462), gp75 ^{TRP1} (Wang et al., J. Exp. Med. 181 (1995) 799-804) and TRP-2 (Wang et al., J. Exp. Med. 184 (1996) 2207-2216).

As a foreign antigen in the sense of the invention any antigen that is capable of causing an immune reaction in the patient's body can be used. Suitably, bacterial or animal antigens are used. In a preferred embodiment, for instance bacterial antigens (e.g., β-galactosidase), bacterial resistance genes (e.g., neo, amp, Kan, tet), or animal antigens, viral antigens, such as HSV-tk, can be used.

In a preferred embodiment, a suicide gene is used as the foreign antigen. The advantage offered by the use of such a suicide gene is that the activation of this gene can be controlled by systemically administering a further substance such as, for instance, cyclosporin or 5'-fluorocytosin. By a "suicide gene" is to be understood a gene which leads, as a result of its expression being influenced from outside, to events taking place within cells, leading to apoptosis. A suicide gene preferably is a gene which, when a chemical substance is applied, preferably systemically, will cause activation of this gene and will produce a substance leading to apoptosis of the cell. An example of a suicide gene is the "Tk gene".

By a "Tk gene" according to the invention the suicide gene thymidine kinase as is described, for instance, in Moolten, F.L., et al., J. Natl. Cancer Inst. 82 (1990) 297-300; Ezzeddine, Z.T., et al., New Biol. 3 (1991) 608-614; and Borrelli, E., et al., Proc. Natl. Acad. Sci. USA 85 (1988) 7572-7576 is to be understood. Other suicide genes such as, for instance, the cytosine deaminase gene which confers lethal sensitivity to 5-fluorocytosine (Mullen, C.A., et al., Proc. Natl. Acad. Sci. USA 89 (1992) 33-37) are also suitable. The HSV-Tk gene is an example of a "suicide" or drug-susceptibility gene. Cells transduced with this gene become sensitive to the antiviral drug ganciclovir (GCV) while non-transduced cells are unaffected. GCV is converted by the HSV-Tk gene to a nucleotide-like precursor that, following further phosphorylation, is incorporated into the DNA of dividing cells and leads to the arrest of the DNA synthesis and cell death.

Further suicide genes are also useful according to the invention, preferably if they can act according to the above-described mechanism. Such suicide gene systems have been described by Bailey, S.M., et al., Gene Therapy 4 (1997) 8081; Gene Therapy 3 (1996) 1143-1150; Kanai, F., et al., Cancer Res. 1 (1997) 461-450; Chiocca, E.A., Clin. Neuro. Surg. 42 (1995) 370-382; Moolten, F.L., Cancer Gene Ther. 1 (1994) 279-287; Blaese, R.M., et al., Eur. J. Cancer 30A (1994) 1190-1193; Mullen, C.A., Pharmacol. Ther. 63 (1994) 199-207; Tiberghien, P., J. Leukoc. Biol. 56 (1994) 203-209.

It is important for the invention that the foreign gene does not act in combination with a drug-susceptible gene, as is described, for instance, by Vile, RG., et al. (1997). It is important that the autologous cell is capable of presenting the tumor antigens on the cell surface. This can only be accomplished if repeated immunization is carried out with tumor cells which are transduced with a foreign gene, or if, preferably, autologous patient cells which are antigen presenting cells and which are transduced with a tumor antigen and a foreign gene are used.

By a "bacterial gene" according to the invention a bacterial gene coding for a protein of a prokaryotic organism is to be understood. Genes conferring resistance to antibiotics, such as, for instance, the neomycin resistance gene or the ampicillin resistance gene, are also preferred. Also preferred are toxin genes from bacteria.

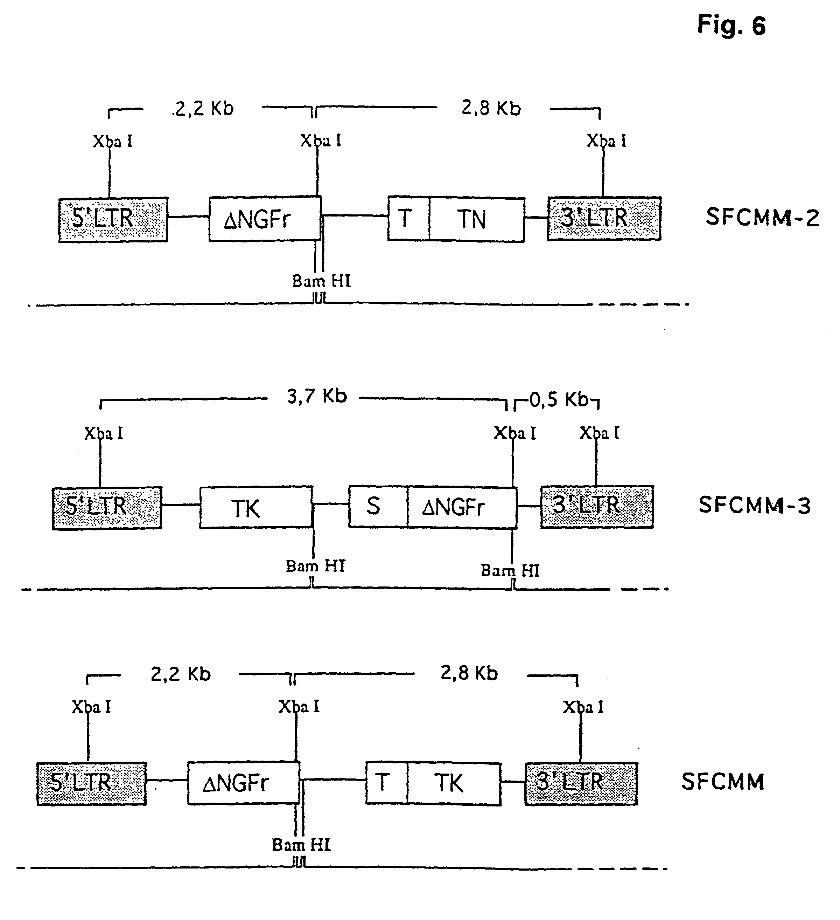

The nucleic acid of the antigen or antigens used according to the invention can be introduced into a eukaryotic expression vector by methods known in the art. Suitable expression vectors are known to a person skilled in the art. Preferably, retroviral vectors are used. Other viral vectors of the state of the art can also be used for the transfer of receptor nucleic acid (e.g., herpes virus, adenovirus, vaccinia viruses). With a certain type of retrovirus, so-called double-copy vectors (Yu, et al., Proc. Natl. Acad. Sci. USA 83 (1989) 3194-3198), a high frequency of rearranged pro-virus and a significantly lower virus titer have been observed. XSN vector viruses (Miller, D.A., et al., Biotechniques 7 (1989) 980-990) are preferred. Typical retroviral vector constructs are shown in Fig. 6.

Expression of proteins of viral or bacterial origin can be strongly immunogenic and can lead to the immune elimination of genetically modified cells.

In allogeneic transplantation a suicide gene can be transduced into BMT-donor lymphocytes, conferring sensitivity to a drug, that may allow the selective elimination of the transduced cells for both therapeutic and safety issues (prevention of graft versus leukemia disease).

Immune recognition and killing of cells transduced by retroviral vectors is a more general phenomenon related to the foreign nature of the proteins expressed by the transduced cells. Indeed, cells expressing a HSV-Tk gene, and preferably, in addition, a gene coding for a bacterial protein like the widely used marker gene neo (neomycin phosphotransferase) are targets of strong immune response, while endogenous proteins (LNGF-R) are not recognized, even if ectopically expressed in a context otherwise extremely immunogenic. Moreover, generation of the specific immune response is completely dependent upon the presence of a functional immune system.

Genetically modified cells are able to induce an immune response when injected into an recipient. TN-specific activity (TN: fusion gene of HSV-tk and neo) was undetectable in unexposed healthy donors including a donor carrying an HLA haplotype identical to the patient. Targets of this response are two proteins: the bacterial product of the neo gene, used as positive-selection-marker in the large majority of gene therapy/marking studies that utilize retroviral vectors, and the product of the HSV-Tk gene and/or the fusion part of the protein. The immunogenicity of the vector-encoded components according to the invention clearly outlines the advantage of this type of gene therapy for the induction of vaccination effects.

In the presence of an active immune system, the schedule of repeated infusions used for the treatment of patients showed the potential to generate a strong and specific immune response. This observation could have important implications in other clinical gene therapy contexts in which immunization is a much wanted effect: Indeed, these results suggest the use of the same approach for protocols of vaccination against well-known antigens such as tumorassociated antigens (Boon, T., et al., 1994), or molecules specifically expressed by infectious agents (Riddell et al., 1996).

For the purposes of this invention, "pharmaceutically acceptable carrier" means any of the standard pharmaceutical carriers. Examples of suitable carriers are well-known in the art and may include, but not be limited to, any of the standard pharmaceutical vehicles, such as a phosphate-buffered saline solution, phosphate-buffered saline containing detergents, preferably nonionic detergents, water, emulsions, such as oil/water emulsions, and various types of wetting agents.

The genetically manipulated cells of this invention may be administered intradermally, subcutaneously, and intramuscularly. Other methods well-known by a person of ordinary skill in the art may also be used.

In order that the antigen presenting cells will be growth-arrested, it is preferred to irradiate these cells or treat them with mitomycin. Such irradiation is carried out preferably at room temperature at a dose of 100 Rad/min. Cells exposed to such irradiation no longer proliferate but are, on account of their mRNA which is still present in the cell, still capable of producing genes, such as cytokines (e.g. IFN-γ, IL-12), when being activated with T cells.

According to the invention, any tumor antigen can be used such as e.g. MAGE, BAGE and GAGE (Van der Bruggen et al., Science 254 (1991) 1643-1647; Boel et al., Immunity 2 (1995) 167-175; Van den Eynde et al., J. Exp. Med 182 (1995) 689-698) or such as e.g. CTL recognizing epitopes from tyrosinase (Brichard et al., J. Exp. Med. 178 (1993) 489-49513), MelanA ^{Mart1} (Coulie et al., J. Exp. Med. 180 (1994) 35-42; Castelli et al., J. Exp. Med 181 (1995) 363-368; Kawakami et al., Proc. Natl. Acad. Sci. USA 91(1991) 3515-3519), gp100^{me17} (Bakker et al., J. Exp. Med. 179 (1994) 1005-1009; Kawakami et al., Proc. Natl. Acad Sci. USA 91 (1994) 6458-6462), gp75 ^{TRP1} (Wang et al., J. Exp. Med. 181 (1995) 799-804) and TRP-2 (Wang et al., J. Exp. Med. 184 (1996) 2207-2216).

It is, however, preferred to use a tumor antigen which is overexpressed in tumor cells of the patient to be treated according to the invention. In order to check this, tumor cells are taken from the patient and it is examined according to methods known to one skilled in the art which of the known tumor antigens is overexpressed.

It is essential for the method according to the invention that the immune response to the vaccine according to the invention should be as strong as possible (optimal). For this purpose, immunization takes place in several cycles. In a preferred immunization protocol, at the first immunization cycle, a high amount of vaccination cells is given. The second and third immunization cycles are carried out after four weeks in each case, followed by further immunizations at two-week intervals. A total of about 10⁵ to 10⁷ T cells/kg body weight or 10⁶ to 10⁸ tumor cells/kg body weight immunizations is preferred, wherein the amount of vaccination cells used should after the second immunization suitably be held at an approximately constant level. The scheme of immunization is essentially dependent on the patient's immune status. If the patient's immunological system is damaged (for example, as a result of irradiation or an immunological disease), an individual optimization of the immunization scheme may be necessary. In any case, an optimum cellular immune response must be ensured. The time at which the second immunization is carried out may be determined by an in vitro assay. To this end, peripheral blood lymphocytes (PBLs) from the patient are incubated in vitro with the vaccination cells and the activation of T cells contained in the PBLs from the patient is determined. Activation of the T cells is suitably carried out by a T cell proliferation assay, e.g., by the determination of the incorporation of ³H-thymidine into the cell. Such methods are known, from the state of the art, to the skilled artisan.

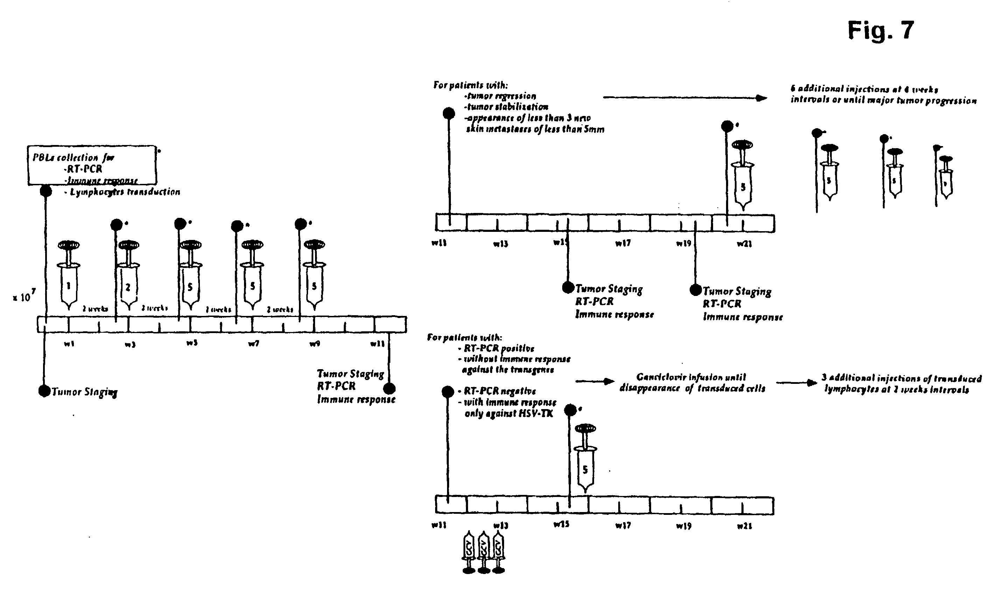

Fig. 7 shows the flow chart of preferred clinical protocols. According to these protocols, it is especially preferred to use according to the invention injections with increasing cell dose (10⁷ to 5x10⁷) at intervals of about two weeks. After about 10 weeks, tumor staging and immune response are tested (for example, RT-PCR). For patients with tumor regression, tumor stabilization and/or appearance of only very few metastases (e.g., less than three new skin metastases of less than 5 mm) six additional injections at four-weeks-intervals or until major tumor progression are given. For the further prosecution, tumor staging and immune response are monitored at about weeks 15 and 19. For patients with positive RT-PCR without immune response against the transgenes or with immune response only against HSV-tk, ganciclovir is given (preferably by infusion) until disappearance of transduced cells. Further additional injections (preferably about three injections) of transduced lymphocytes are given at preferred intervals of two weeks.

As mentioned above, it is preferred to use vectors in which, besides a suitable tumor antigen, also a suicide gene (preferably, HSV-I-tk) is applied. The advantages derived from the additional use of HSV-I-tk are:
- safety;
- internal control of vaccination effectiveness (immunocompetent patients specifically respond to HSV-I-Tk and Neo);
- helper effect for the presentation of the Tum-Ags (specific response against neo and hygB always observed in the presence of HSV-I-Tk);
- GCV-mediated increase of the immune response (killing of transduced T blasts by GCV can induce presentation of the Tum-Ags by auto-APC).

The following examples, references and drawings are provided to aid the understanding of the present invention, the true scope of which is set forth in the appended claims. It is understood that modifications can be made in the procedures set forth without departing from the spirit of the invention.

### Figure Legends

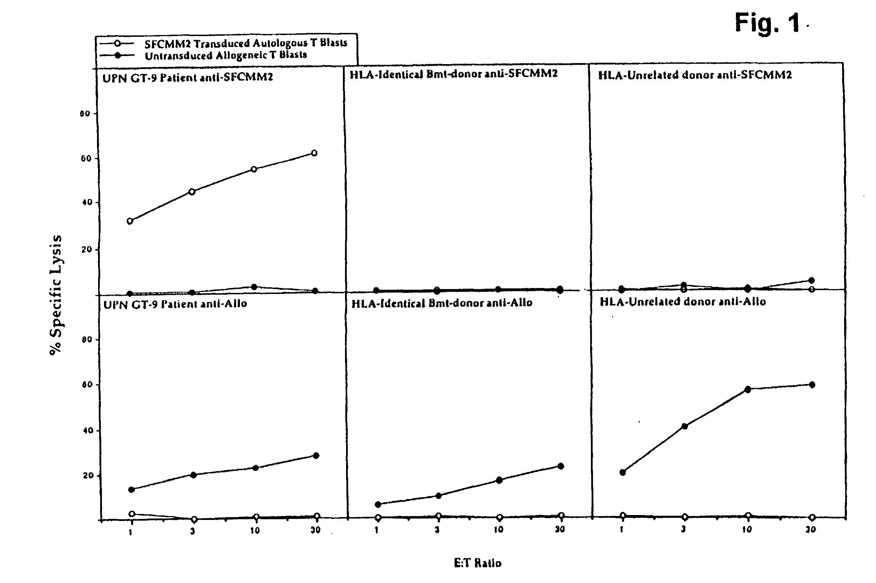

- **Figure 1**: Lytic activity specific for the SFCMM2-transduced cells by PBMC from UPN GT-9 patient. PBMC isolated from UPN GT-9 patient, BMT-donor (bone marrow transplant donor) and a HLA-unrelated donor were stimulated with autologous SFCMM2-transduced T blasts (upper panels) and with allogeneic T blasts (lower panels). On day 15 the lytic activities of the lymphocytes were measured against autologous-transduced and allogeneic T blasts in a standard chromium release assay at the indicated E/T ratios.

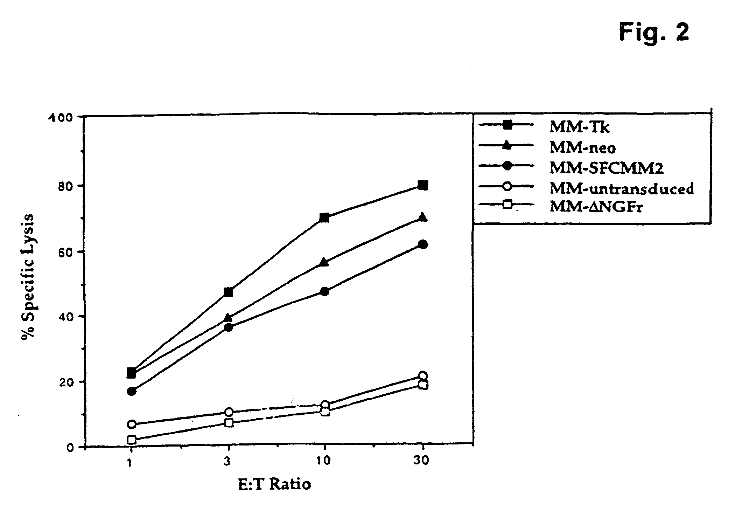

- **Figure 2**: HSV-Tk and neo are the target antigens of the SFCMM2-specific immune response. An EBV cell line from the BMT-donor (MM LCL) was transduced with retroviral vectors encoding for single components of the SFCMM2 vector and used as target in a standard chromium release assay at the indicated E/T ratios.

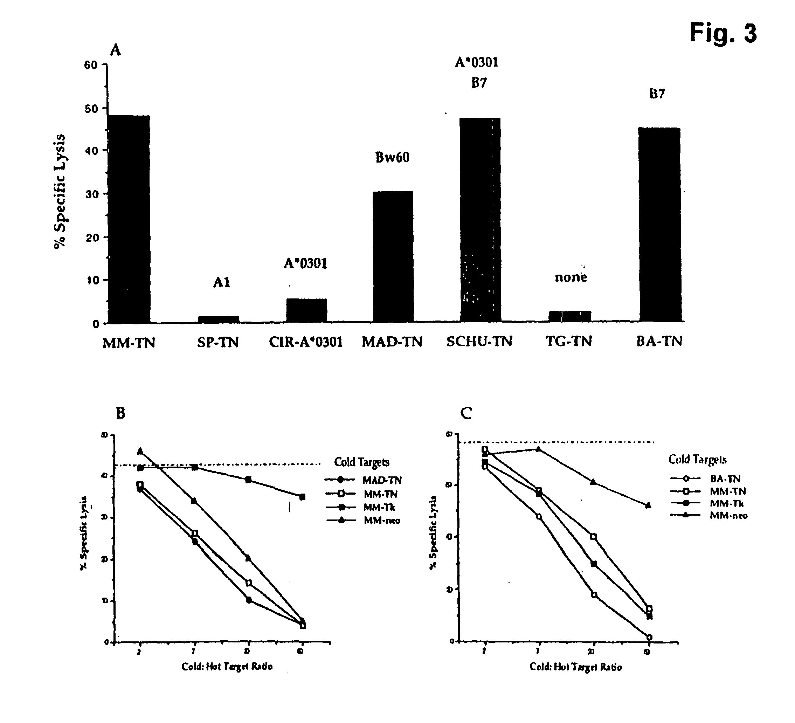

- **Figure 3**: HLA-Bw60 and -B7 are the restriction elements responsible of HSV-Tk and neo presentation respectively. (A) Lysis by SFCMM2-specific TCL from UPN GT-9 patient of matched and mismatched LCL targets transduced with the SFCMM2 vector encoding for the TN fusion protein. (B) Lysis of MAD-TN (HLABw60), was significantly inhibited by HLA-Bw60 LCL (MM LCL and MAD LCL) expressing TN and neo, but not Tk alone. The arrow indicates the level of lysis of MAD-TN without cold targets. (C) Lysis of BA-TN (HLA-B7) was significantly inhibited by HLA-B7 LCL (MM LCL and BA LCL) expressing TN and Tk, but not neo alone. The arrow indicates the level of lysis of BA-TN without cold targets.

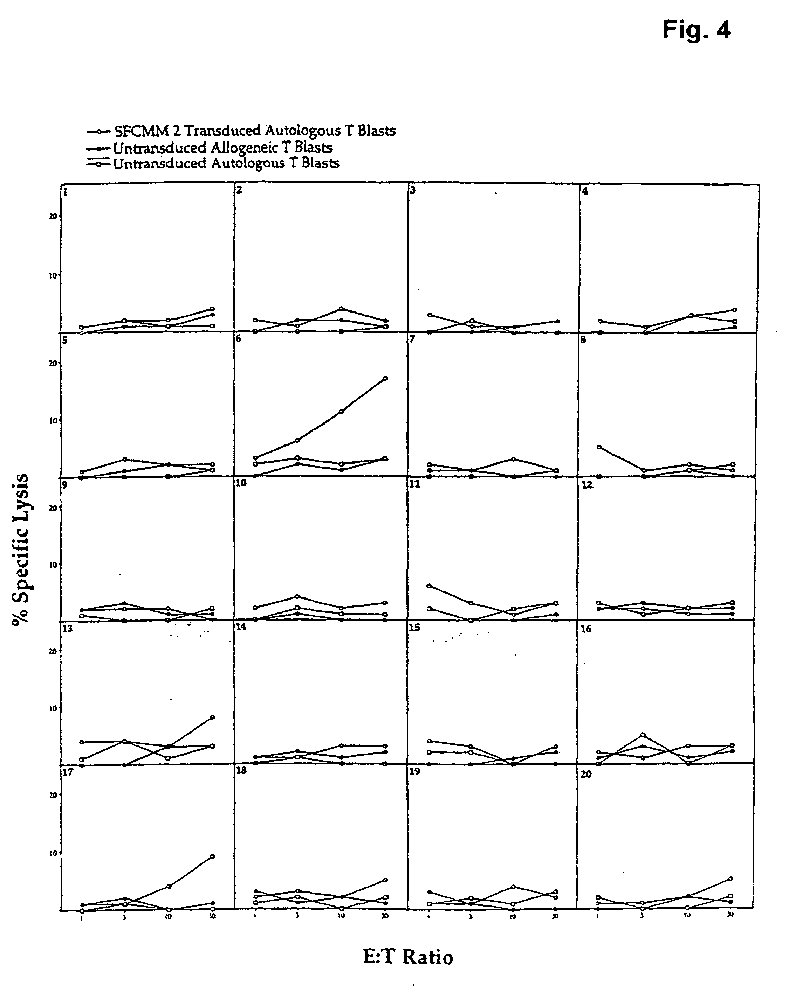

- **Figure 4**: TN-specific effectors are present at low frequency also in a healthy donor. PBMC containing 2x10⁶ CD3+ lymphocytes, isolated from BMT-donor were stimulated, in twenty independent microcoltures, with autologous SFCMM2-transduced T blasts. On day 10 the lytic activities of the lymphocytes were measured against autologous-transduced and allogeneic T blasts in a standard chromium release assay at the indicated E/T ratios.

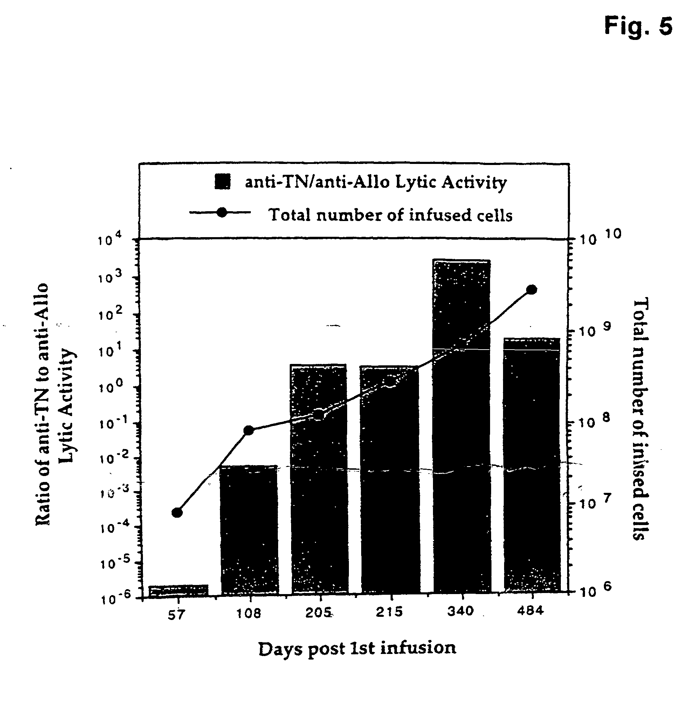

- **Figure 5**: Correlation between development of TN-specific response and the injection of SFCMM2-transduced lymphocytes. Samples 1 to 5 were collected from patient UPN GT-9, at different time after BMT transplantation. PBMC containing 10⁶ CD3+ lymphocytes from each sample were stimulated with allogeneic and autologous SFCMM2-transduced T blasts and tested 10 days later for their lytic activity against autologous-transduced and allogeneic T blasts. Results are plotted as TN/Allo ratio, that is the ratio between the anti-TN lytic units/10⁶cells to the anti-Allo lytic units/10⁶ cells. Number of infused cells at the different time is reported on the right ordinate axis.
- **Figure 6**: Proviral structure of three preferred retroviral vectors. ΔNGFR: LNGFR cDNA, deleted of intracellular domain. T: HSV-tk promoter. TN: HSV-tk/Neo fusion gene. TK: wild-type HSV-tk gene. S: SV40 early promoter. The positions of restriction sites used for Southern blot analysis with the size of ejected fragments is also shown.
- **Figure 7**: Preferred clinical protocols (flow chart).

### Example 1

### Retroviral vectors

Three different vectors (Fig. 6) were derived from the LXSN backbone (Miller, D.A., et al., Biotechniques 7 (1989) 980-990). SFCMM-2 was obtained by cloning the 0.95 kb fragment from the human LNGFR cDNA in the HpaI site of LXSN, while the XcaI/NcoI fragment from TNFFus69 plasmid (Schwartz, F., et al., Proc. Natl. Acad. Sci. USA 88 (1991) 10416-10420), containing the HSV-tk promoter and the tk/neo fusion gene was cloned in place of the SV40 promoter/neo (Hind III/NaeI fragment) in LXSN. SFCMM was derived from SFCMM-2 by replacing the BglII/NaeI and NaeI/NaeI fragments containing the tk/neo gene, with the HSV-tk BamHI/EcoRI 1150 bp fragment from pBTK3.3. SFCMM-3 was derived from the LXSN by replacing the HindIII/NaeI and NaeI/NaeI fragments, containing the neoR gene, with the 0.95 kb fragment from the human LNGFR cDNA, and by cloning the HSV-tk BamHI/EcoRI 1150 bp fragment from pBTK3.3 in the HpaI site in LXSN, under LTR transcriptional control. Vector DNAs were converted to corresponding viruses by the transinfection protocol. Briefly, vector DNA was transfected in the E86 ecotropic packaging cell line by standard calcium-phosphate co-precipitation (Sambrook, J., et al. (1989) Molecular cloning: A laboratory manual, Cold Spring Harbor Laboratory Press, New York, USA). 24 h after transfection, residual DNA precipitate was removed, and fresh medium added; 48 h later, E86 supernatants were harvested and used to infect the amphotropic cell line AM12 (Miller, D.A., et al. (1989)) for 16 h in the presence of 8 µg/ml polybrene. Transduction of AM12 cells could be detected by FACS analysis with a murine anti-LNGFR antibody. Infected AM12 cells were either selected in G418 or immunoselected; G418 selection was performed in Dulbecco's modified Eagle's medium (DMEM; GIBCO, Grand Island, NY, US), supplemented with 10% fetal calf serum (FCS; Hyclone, Logan, UT, US) and containing 0.8 mg/ml G418 (Boehringer Mannheim GmbH, DE); immunoselection was performed by incubating the cells with the murine antihuman LNGFR monoclonal antibody and subsequent sorting with goat anti-mouse-IgG-coated magnetic beads (Dynabeads M-450, Dynal A.S., NO).

### DNA analysis

High molecular weight genomic DNA was obtained from cells by standard phenol/chloroform extraction (Sambrook (1989)), digested to completion with appropriate digestion enzymes (Boehringer Mannheim GmbH, DE), electrophoresed in 0.7% agarose gel in Tris-acetate-EDTA buffer, transferred to a nylon membrane (Hybond-N, Amersham, UK) by Southern capillary blotting and hybridized to 10⁷ dpm of ³²P-labelled probe. DNA probes were the HSV-tk fragment (see above) and the ΔLNGFR cDNA. Filters were washed under high stringency conditions and exposed to Amersham Hyperfilm-MP films (Amersham, UK) at -80°C.

### Cell surface phenotyping

Cell surface expression of ΔLNGFR was monitored by flow cytometry using a murine antihuman LNGFR monoclonal antibody with an indirect fluorescence labelling method.

### Stimulator and target cells:

In order to test immunity against genetically modified cells, the following cell lines were utilized as stimulator and target cells. T blasts were obtained by cultivation of PBMC, isolated by Lymphoprep (Nycomed, Oslo, Norvay) density-gradient centrifugation, in the presence of 1 βg/ml ofPHA (Boehringer Mannheim GmbH, Germany) and 100 U/ml recombinant human IL2 (Chiro, Milano, Italy). B-lymphoblastoid cell lines (LCL) were derived by transformation of peripheral blood B lymphocytes with the B95-8 strain of EBV. The LCLs Madura and Schu were obtained from the American Type Culture Collection (ATCC, Rockville, MD). A summary of the cell lines used and their HLA haplotypes are listed in Table 1. LCL and T blasts were obtained and grown in IMDM (GIBCO, Grand Island, NY) supplemented with 10% FCS (Hyclone, Logan, UT) or 10% human serum respectively.

### Retroviral transduction of stimulator and target cells:

Genetically modified T cells and LCL utilized as stimulators and/or targets in the ex vivo studies were transduced by co-culture with packaging cell lines producing the SFCMM2 vector. Retroviral vectors encoding for single components of SFCMM2 has been described in WO 95/06723. PBMC were stimulated by culture in the presence of PHA (1 pg/ml) and r-hu-IL2 (100 U/ml). Viral infection was performed by cocultivation of stimulated PBMC on a monolayer of irradiated (10,000 rads) packaging cell lines in the presence of polybrene (8Rg/ml). After 72 hours lymphocytes were harvested and seeded in fresh medium (10⁶/ml). The percentage of infected cells was evaluated 48 hours later by flow cytometry for NGF-R expression with a murine antihuman LNGFR monoclonal antibody. The NGF-R positive cells were purified by magnetic cell sorting with Rat anti-mouse-IgGI coated beads (Dynabeads M-450, Dynal AS. N0212 Oslo, Norway). In the same way, pure populations of retrovirus infected LCL were obtained by cocultivation with packaging cell lines and magnetic cell sorting.

### Establishment of TN-specific T cell lines and clones:

PBMC or CD3+ lymphocytes (1x10⁶) from patient UPN GT-9 and healthy donors were cultured with irradiated (5000 rads) autologous (HLA-identical for UPN GT-9 stimulations) SFCMM2-infected T lymphocytes and with allogeneic T blasts, in 24 well plates. On day 3 a final concentration of 10 U/ml of IL2, was added to each culture. Cytotoxic T lymphocytes were maintained in culture by weekly stimulations in the same conditions. The cytolytic activity was evaluated about 10 days after the stimulation. CTL clones were obtained by culturing T cell lines after 2 rounds of stimulation, in 96 well-plates at limiting numbers with 10⁴ irradiated (5,000 rads) SFCMM2-infected T lymphocytes and 2x10⁴ irradiated (10,000 rads) allogeneic LCL as feeders, in the presence of IL2 (50 U/ml). After 7 days the clones were stimulated in the same conditions and tested for specific lysis one week later.

### Assay for cytolytic activity:

Lysis of target cells by T cell lines and clones was tested by chromium release assay as previously described (Herin, M., et al., 1987). Effector cells and 2000 51Cr-labeled targets were incubated at various ratios in 96 conical microplates. Chromium release in the supernatant was measured after 4 h of incubation. Lytic units were calculated as the number of effectors/10⁷ cells required to obtain 30% target lysis, with 2000 labelled target cells being used in the assay. For cold-target inhibition tests, serial dilutions of unlabeled targets, starting from 1:60 to 1:1, cold:hot target cell ratio, were added to the effector cells and incubated for 60 minutes at 37°C prior to addition of the 51Cr-labeled targets.

### Example 2

### Patient characteristics:

UPN (Patient Unique Patient Number) GT-9 is a 52-year-old man who received an allogeneic bone marrow transplantation (BMT) from his HLA-identical brother in first chronic phase, he relapsed six months later, with CML in chronic phase. In an attempt to reinduce complete remission, he was treated with escalating doses of donor lymphocytes. In order to control the risk of GVHD associated with this approach, BMT-donor lymphocytes were transduced with a retroviral vector coding for a truncated form of the low affinity human nerve growth factor receptor (LNGF-R), and for the HSV-Tk/neo (TN) fusion protein, a bifunctional protein carrying both the HSV-Tk activity and the neomycin resistance. The presence of the LNGF-R on the cell surface allows the in vivo follow-up of the infused BMT-donor lymphocytes, while TN expression confers sensitivity to the drug ganciclovir, necessary for the in vivo specific elimination of the transduced cells. After the infusion of a total number of 7x10⁷ BMT-donor lymphocytes, his blood count normalized and the disease stabilized. Four months after the first infusion, he developed acute hepatic GVHD that resolved after ganciclovir treatment. After full regression of GVHD symptoms and signs, in an attempt to achieve complete remission, the patient received five additional infusions, in escalating doses (from 10⁵/kg to 3x10⁷/kg), to a total of 3x10⁹ SFCMM2-modified cells. However, this aggressive therapeutic approach produces no improvement of the disease nor recurrence of GVHD. In addition, genetically-modified cells in bone marrow and in the circulation became nearly undetectable by both FACS analysis and PCR. All these data taken together show the immune elimination of transduced cells.

### Example 3

### Detection and characterization of the transgene-specific immune response:

In order to document and characterize in vitro the anti-BMT-donor immune response, PBMC were isolated from patient UPN GT-9 after he received 3x10⁹ transduced BMT-donor lymphocytes in eight infusions in escalating doses and were stimulated in vitro by irradiated SFCMM2-transduced autologous T blasts, in the presence of IL-2. Similarly, HLA-identical BMT-donor cells and cells from an HLA-unrelated control were stimulated with SFCMM2-transduced autologous T blasts, and analyzed in parallel. The lytic activity of the responder cells against the stimulators was determined after one and two rounds of stimulation. No difference was observed between the two time points. Under these conditions, cytotoxic T cells with specificity for the SFCMM2-transduced targets were exclusively obtained from patient UPN GT-9 (Fig. 1). No such cytotoxic T cells could be established from appropriate controls including an unrelated control and the BMT-donor. To assess the patient's and control's immunocompetence, specific immune response against allogeneic targets (in Fig. 1, lower panels, indicated as: "Allo") was tested and detected in all the responders (Fig. 1). Most relevant, the lytic activity of the SFCMM2-specific T cells was restricted to the genetically-modified cells, as demonstrated by resistance of the untransduced BMT-donor T targets to lysis, and was therefore specific for products encoded by the SFCMM2 vector.

### Relative immunogenicity of the different transgenes:

To investigate whether the TN fusion protein, or the LNGF-R, were directly involved in the generation of the response, a LCL (MM in Table 1) derived from the BMT-donor was separately transduced by different retroviral constructs encoding for single components of the SFCMM2 vector. After purification by magnetic beads, pure populations of transduced LCL cells were obtained and used as target cells in a standard cytotoxicity assay. The effector cells specifically recognized both the components of the TN fusion protein separately (Fig. 2). LCL expressing the suicide HSV-Tk gene or the bacterial neo gene were lysed at the same level.

On the contrary, LCL expressing the LNGF-R were not recognized at all (Fig. 2). SFCMM2-specific effector cells with identical activity were obtained from patient UPN GT-9 in several independent experiments. After two rounds of restimulation in bulk culture one of the specific T cell line was cloned by limiting dilution to test the frequency of individual specificities. Eight out of the 98 clones tested (8.2%) recognized the HSV-Tk and 26 (26.5%) the neo gene product, suggesting that the anti-neo response plays a major role in the phenomenon.

The relevance of the anti-Tk versus the anti-neo response in the in vivo priming of the anti-TN immunity may have a crucial role in defining new therapeutic strategies. In vitro stimulation with HSV-1-infected PBMC is known to induce a strong cytotoxic and proliferative response only from HSV-1 immune individuals (Maccario, R, et al., 1992). High frequencies of HSV-specific CTL both CD4-positive (Schmid, D.S., and Mawle, A.C., 1991), and CD8-positive (Maccario et al., 1992; Yasukawa, M., and Zarling, 1985) have been described in immune donors. The observation that UPN GT-9 patient was HSV-seropositive suggests that anti-HSV-precursors could be responsible for his in vivo response against the HSV-Tk expressed by the transduced cells. It is well known that expression of strong antigens, able to activate a local release of cytokines, ensures an immune response against the weaker antigens present on the same cell (Boon et al., 1994). Therefore, the anti-Tk response might be actively involved in the specific priming against neo. HSV-Tk has not yet been described as target of the cellular mediated response during HSV-1 infection. Additionally non-structural immediate-early proteins were shown to be the major CTL targets in murine models (Martin, S., et al., 1988).

Following this observation, the analysis of immunity against the transgene to patients involved in other gene therapy trials was extended. The neo-gene in particular is widely used in these studies including an ADA gene therapy clinical study (Bordignon, C., et al., 1995). Following the same detection strategy described in this study, any immunity against neo in ADA gene therapy patients was failed to be detected. These data confirm the potential role of the immune status of the recipient at the time of exposure to the transgene.

### MHC-restriction of immune recognition:

Due to short term life of CTL clones, further experiments were performed with polyclonal T cell lines. After repeated rounds of stimulation lysis was HLA-class I restricted as demonstrated by specific inhibition of lytic activity by anti-HLA-A,-B,-C (W6/32) or anti-HLA-B,-C (4E) antibodies. Further characterization of the MHC-restriction elements, was obtained using a panel of HLA-A,-B-matched and -mismatched targets (Table 1).

**Table 1**

| **Description of the LCL used as targets** | | | |
|---|---|---|---|
| **Cell line** | **Type** | **HLA** | **Source** |
| MM | B95-8 transformed LCL from MM BMT-donor | A*0301, A1; Bw60, B7; Cw3 | |
| TG | B95-8 transformed LCL from TG donor | A24, A11; B15, B49 | |
| SP | B95-8 transformed LCL from SP donor | A1, A32; B44, B8; Cw4, Cw6 | |
| BA | B95-8 transformed LCL from BA donor | A24, A29; B7, B63; Cw7 | |
| MADURA | LCL | A2; Bw60; C10 | ATCC |
| SCHU | LCL | A*0301; B7; Cw7 | ATCC |
| C1R-A*0301 | LCL transfected with A*0301 | A*0301; B35; Cw4 | C. Castelli |

Figure 3 panel A shows that after transduction with the SFCMM2 vector encoding for the fusion-protein TN, only targets expressing B7 or Bw60 were lysed. Lysis of the HLA-B7 BA-TN is inhibited by the addition of high concentration of cold MM LCL expressing the TN fusion protein or the HSV-Tk alone, but not the neo gene alone (Fig. 3C). The contrary was observed with the HLA-Bw60 MADURA-TN, whose lysis was efficiently inhibited by the MM-neo LCL (Fig. 3B). These results demonstrate that the genetically modified cells are recognized in a HLA-B7, -Bw60 fashion and strictly suggests that B7 is the restriction element responsible for the HSV-Tk presentation, while Bw60 is predominantly involved in the neo recognition. In spite of the intensity of the immune response observed (1 round of stimulation is enough to induce a fully specific population of effector cells), the effector cell lines are probably oligoclonal, as suggested by the low number of HLA-class I alleles involved in antigen presentation. Only 2 out of the 4 investigated alleles have a role in antigen recognition and antigenic peptides presented by HLA-A alleles have not been detected.

### Kinetic of the in vivo priming:

To investigate whether the failure to induce a TN-specific CTL response from healthy donors could be explained by the absence of the relevant CTL precursors in the culture (Cerny, A., et al., 1995), 20 replicates each containing 2x10⁶ CD3+ lymphocytes from the BMT-donor were stimulated under the conditions previously described. After one week of stimulation, culture No. 6 specifically recognized the autologous SFCMM2-transduced cells (Fig. 4), demonstrating that the absence of specific response previously observed in single culture from normal PBMC is probably due to the low frequency of T cell precursors.

Based on the results of this experiment and on the observation that TN-specific effectors can be regularly expanded from low number of PBMC from patient UPN GT-9, it can be concluded that this immune reactivity is a consequence of the injection of the transduced cells, that specifically stimulated his immune system in a vaccine-like mode.

To further investigate the correlation between injection and immune response against genetically modified cells, UPN GT-9 PMBC collected at different times during this treatment was stimulated. Indeed, variations in the amount of the TN-specific response between the different samples could simply reflect the immunological status of the patient through disease progression or changes in chemotherapy. Moreover, the level of the immune response could be affected by random factors occurring in the cryopreservation of the samples that might influence viability of the stored cells. To avoid these artifacts and the experimental variability, the same number of CD3+ cells from each samples both against allogeneic and SFCMM2-autologous T blasts were stimulated. The anti-TN response detected was plotted as ratio between anti-TN and anti-Allo lytic activity from the same sample, to allow a comparison of the responses during the therapy. Moreover, the lytic activity of the cultures was evaluated almost exclusively after only 1 round of stimulation, when it is still proportional to the number of cytolytic antigen-specific T cell precursors present in the starting samples (Lucas, K.G., et al., 1996).

As expected, a continuous increase of the amount of TN-reactive T cell precursors was observed (Fig. 5): the lytic activity was undetectable on day 57 since first infusion (after the injection of 9x10⁶ SFCMM2-transduced cells), and reached a maximum on day 340 (after the injection of a total of 7x10⁸ SFCMM2-transduced cells). The lack of anti-TN reactivity at day 57 was probably due to the low level of in vivo antigenic stimulation, due both to the low number of injected cells and to the immunoincompetence status of the patient in the early post-transplant period (Lucas et al., 1996). The last point was confirmed by the observation that the anti-allo response at day 57 could be detected only after two rounds of in vitro stimulation. The increments of the lytic activity observed in the cultures from days 205 and 340 could find an explanation in the increased number of injected cells. However, an alternative explanation should be considered: on day 150 the patient developed acute GVHD, ganciclovir was administered and GVHD resolved promptly. It is well known in some murine as well as human tumor systems that tumor cell disruption mediated by ganciclovir plays a major role in the induction of a tumor-specific immune response, probably accounting for the presentation of some tumor proteins by professional APC in the context of it own MHC class I and II complex (Vile and Russell, 1994; Barba, D., et al., 1994). Even if recent reports (Gagliardi, M.C., et al., 1995) suggest that activate T lymphocytes are themselves able to induce a Th-independent cytolytic T cell response, immunogenicity of the TN protein released by the transduced lymphocytes could be significantly enhanced by APC presentation.

### List of References

Anderson, W.F., Science 256 (1992) 808-813
Bailey, S.M., et al., Gene Therapy 3 (1996) 1143-1150
Bailey, S.M., et al., Gene Therapy 4 (1997) 8081
Bakker et al., J. Exp. Med. 179 (1994) 1005-1009
Barba, D., et al., Proc. Natl. Acad. Sci. USA 91 (1994) 4348-4352
Blaese, R.M., et al., Eur. J. Cancer 30A (1994) 1190-1193
Boel et al., Immunity 2 (1995) 167-175
Boon, T., et al., Annu. Rev. Immunol. 12 (1994) 337-365
Bordignon, C., et al., Science 270 (1995) 470-475
Borrelli, E., et al., Proc. Natl. Acad. Sci. USA 85 (1988) 7572-7576
Brichard et al., J. Exp. Med. 178 (1993) 489-49513
Castelli et al., J. Exp. Med 181 (1995) 363-368
Cerny, A., et al., Eur. J. Immunol. 25 (1995) 627-663
Chiocca, E.A., Clin. Neuro. Surg. 42 (1995) 370-382
Colombo, M.P.a.F.G., Immunol. Today 15 (1994) 48-51
Coulie et al., J. Exp. Med. 180 (1994) 35-42
Cullis, J.O., et al., Blood 79 (1992) 1379-1381
Ezzeddine, Z.T., et al., New Biol. 3 (1991) 608-614
Gagliardi, M.C., et al., Int. Immunol. 7 (1995) 1741-1752
Gansbacher, B., The Mount Sinai J. Med. 61 (1994) 301-309
Helslop, H.E., et al., Nature Med. 2 (1996) 551-555
Herin, M., et al., Int. J. Cancer 39 (1987) 390-396
Kanai, F., et al., Cancer Res. 1 (1997) 461-450
Kawakami et al., Proc. Natl. Acad Sci. USA 91 (1994) 6458-6462
Kawakami et al., Proc. Natl. Acad. Sci. USA 91 (1991) 3515-3519
Kolb, H.J., et al., Blood 76 (1990) 2462-2465
Lucas, K.G., et al., Blood 87 (1996) 2294-2603
Maccario, R., et al., Viral Immunol. 5 (1992) 93-103
Martin; S., et al., J. Virol. 62 (1988) 2265-2273
Miller, A.D., Nature 357 (1992) 455-460
Miller, D.A., et al., Biotechniques 7 (1989) 980-990
Moolten, F.L., Cancer Gene Ther. 1 (1994) 279-287
Moolten, F.L., et al., J. Natl. Cancer Inst. 82 (1990) 297-300
Mullen, C.A., et al., Proc. Natl. Acad. Sci. USA 89 (1992) 33-37
Mullen, C.A., Pharmacol. Ther. 63 (1994) 199-207
Pardol, M.D., Current Opinion in Immunol. 5 (1993) 719-725
Riddell, S.R., et al., Nature Med. 2 (1996) 216-223
Rosenberg, S., Immunol. Today 9 (1988) 58-62
Rosenberg, S.A., et al., N. Engl. J. Med. 139 (1988) 1676-1680
Rosenberg, S.A., et al., N. Engl. J. Med. 323 (1991) 570-578
Rosenberg, S.A., J. Clin. Oncol. 10 (1992) 180-199
Sambrook, J., et al. (1989) Molecular cloning: A laboratory manual, Cold Spring Harbor
Laboratory Press, New York, USA
Schmid, D.S., and Mawle, A.C., Reviews of Infect. Diseases 13 (1991) 946-949
Schwartz, F., et al., Proc. Natl. Acad. Sci. USA 88 (1991) 10416-10420
Tiberghien, P., J. Leukoc. Biol. 56 (1994) 203-209
Van den Eynde et al., J. Exp. Med 182 (1995) 689-698
Van der Bruggen et al., Science 254 (1991) 1643-1647
Van Rhee, F., et al., Blood 83 (1994) 3377-3383
Vile, R., and Russell, S.J., Gene Therapy 1 (1994) 88-98
Vile, R.G., et al., International Journal of Cancer 71 (1997) 267-274
Wang et al., J. Exp. Med. 181 (1995) 799-804
Wang et al., J. Exp. Med. 184 (1996) 2207-2216
Williams Hematology, eds. E. Beutler et al., 5th edition (1979) McGraw-Hill, pp. 1611-1617 WO 95/06723
Yang, Y., et al., J. Virol. 69 (1995) 2004-2015
Yasukawa, M., and Zarling, J.M., J. Immunol. 134 (1985) 2679-2682
Yu, et al., Proc. Natl. Acad. Sci. USA 83 (1989) 3194-3198

## Claims

1. Use of an autologous or HLA-related antigen presenting cell (APC) which is capable of presenting an antigen in a patient, said cell being transduced with a tumor antigen and a foreign antigen capable of causing an immune reaction in a patient's body, for the preparation of a medicament for at least two subsequent administrations for tumor vaccination.

2. Use according to claim 1 wherein the cell is a T cell.

3. Use according to claims 1 or 2 wherein cell is transduced with a viral thymidine kinase gene.

4. Use according to claim 3 wherein the viral thymidine gene is an HSV-Tk gene.

5. Use according to any preceding claim wherein the cell is transduced with a bacterial resistance gene.

6. Use according to claim 5 wherein the bacterial gene codes for neomycin resistance.

7. Use according to any preceding claim wherein the tumor antigen is selected from MAGE, BAGE, GAGE, a CTL-recognizing epitope from tyrosinase, MelanA^{Mart1}, gp100^{mel7}, gp75^{TRP1} or TRP-2.

8. Use according to any preceding claim wherein the cell is transduced in vitro.

9. Use according to any preceding claim wherein the medicament is used for the treatment of neuroblastoma or a brain tumor.

10. Use according to any preceding claim further comprising the use of a cytokine to cause local inflammation.

11. Use according to any preceding claim wherein the transduction is carried out locally, systemically or ex vivo.

12. A method for preparing an APC as defined in preceding claim comprising transducing ex vivo the APC with a tumor antigen and a foreign antigen capable of causing an immune reaction in a patient's body.

13. A method according to claim 12 wherein the APC is a T cell.

14. An antigen presenting cell transduced with a tumor antigen and a foreign antigen capable of causing an immune reaction in a patient's body.

15. A cell according to claim 14 wherein the cell is a T cell.

16. A cell according to claims 14 or 15 wherein cell is transduced with a viral thymidine kinase gene.

17. A cell according to claim 16 wherein the viral thymidine gene is an HSV-Tk gene.

18. A cell according to any one of claims 14 to 17 wherein the cell is transduced with a bacterial resistance gene.

19. A cell according to claim 18 wherein the bacterial gene codes for neomycin resistance.

20. A cell according to any one of claims 14 to 19 wherein the tumor antigen is selected from MAGE, BAGE, GAGE, a CTL-recognizing epitope from tyrosinase, MelanA^{Mart1}, gp100^{mel7}, gp75^{TRP1} or TRP-2.

## Patentansprüche

1. Verwendung einer autologes oder HLA-verwandtes Antigen präsentierenden Zelle (APC), welche in der Lage ist, ein Antigen in einem Patienten zu präsentieren, wobei die Zelle mit einem Tumorantigen und einem fremden Antigen, das in der Lage ist, eine Immunreaktion in einem Patientenkörper zu verursachen, transduziert ist, für die Herstellung eines Medikaments für wenigstens zwei aufeinanderfolgende Verabreichungen für eine Tumorimpfung.

2. Verwendung nach Anspruch 1, wobei die Zelle eine T-Zelle ist.

3. Verwendung nach Anspruch 1 oder 2, wobei die Zelle mit einem viralen Thymidinkinasegen transduziert ist.

4. Verwendung nach Anspruch 3, wobei das virale Thymidingen ein HSV-Tk-Gen ist.

5. Verwendung nach einem der vorangegangenen Ansprüche, wobei die Zelle mit einem bakteriellen Resistenzgen transduziert ist.

6. Verwendung nach Anspruch 5, wobei das bakterielle Gen Neomycin-Resistenz codiert.

7. Verwendung nach einem der vorangegangenen Ansprüche, wobei das Tumorantigen unter MAGE, BAGE, GAGE, einem CTL-erkennenden Epitop von Tyrosinase, MelanA^{Mart I}, gp100^{mel7}, gp75^{TRP1} oder TRP-2 ausgewählt ist.

8. Verwendung nach einem der vorangegangenen Ansprüche, wobei die Zelle in vitro transduziert wird.

9. Verwendung nach einem der vorangegangenen Ansprüche, wobei das Medikament für die Behandlung von Neuroblastom oder einem Gehirntumor verwendet wird.

10. Verwendung nach einem der vorangegangenen Ansprüche, welche weiterhin die Verwendung eines Cytokins zur Verursachung einer örtlichen Entzündung umfaßt.

11. Verwendung nach einem der vorangegangenen Ansprüche, wobei die Transduktion lokal, systemisch oder ex vivo durchgeführt wird.

12. Verfahren zur Herstellung einer APC, wie sie in den vorangegangenen Ansprüchen definiert ist, welches das Transduzieren der APC mit einem Tumorantigen und einem fremden Antigen, das in der Lage ist, eine Immunreaktion in einem Patientenkörper zu verursachen, umfaßt.

13. Verfahren nach Anspruch 12, wobei die APC eine T-Zelle ist.

14. Antigen präsentierende Zelle, die mit einem Tumorantigen und einem fremden Antigen, welches in der Lage ist, eine immunreaktion in einem Patientenkörper zu verursachen, transduziert ist.

15. Zelle nach Anspruch 14, wobei die Zelle eine T-Zelle ist.

16. Zelle nach Anspruch 14 oder 15, wobei die Zelle mit einem viralen Thymidinkinasegen transduziert ist.

17. Zelle nach Anspruch 16, wobei das virale Thymidingen ein HSV-Tk-Gen ist.

18. Zelle nach einem der Ansprüche 14 bis 17, wobei die Zelle mit einem bakteriellen Resistenzgen transduziert ist.

19. Zelle nach Anspruch 18, wobei das bakterielle Gen Neomycin-Resistenz codiert.

20. Zelle nach einem der Ansprüche 14 bis 19, wobei das Tumorantigen unter MAGE, BAGE, GAGE, einem CTL-erkennenden Epitop von Tyrosinase, MetanA^{Mart l}, gp100^{mel7}, gp75^{TRP1} oder TRP-2 ausgewählt ist.

## Revendications

1. Utilisation d'une cellule présentatrice d'antigène (CPA), autologue ou en rapport avec HL-A, qui est capable de présenter un antigène chez un patient, ladite cellule étant transduite avec un antigène tumoral et un antigène étranger capable de provoquer une réaction immunitaire dans l'organisme d'un patient, pour la préparation d'un médicament pour au moins deux administrations ultérieures pour une vaccination anti-tumorale.

2. Utilisation suivant la revendication 1, dans laquelle la cellule est un lymphocyte T.

3. Utilisation suivant la revendication 1 ou 2, dans laquelle la cellule est transduite avec un gène de thymidine-kinase viral.

4. Utilisation suivant la revendication 3, dans laquelle le gène de thymidine viral est un gène HSV-Tk.

5. Utilisation suivant l'une quelconque des revendications précédentes, dans laquelle la cellule est transduite avec un gène de résistance bactérien.

6. Utilisation suivant la revendication 5, dans laquelle le gène bactérien code pour la résistance à la néomycine.

7. Utilisation suivant l'une quelconque des revendications précédentes, dans laquelle l'antigène tumoral est choisi entre MAGE, BAGE, GAGE, un épitope de reconnaissance de CTL provenant de la tyrosinase, MelanA^{Mart1}, gp100^{me17}, gp75^{TRP1} et TRP-2.

8. Utilisation suivant l'une quelconque des revendications précédentes, dans laquelle la cellule est transduite in vitro.

9. Utilisation suivant l'une quelconque des revendications précédentes, dans laquelle le médicament est utilisé pour le traitement d'un neuroblastome ou d'une tumeur cérébrale.

10. Utilisation suivant l'une quelconque des revendications précédentes, comprenant en outre l'utilisation d'une cytokine pour provoquer une inflammation locale.

11. Utilisation suivant l'une quelconque des revendications précédentes, dans laquelle la transduction est effectuée de manière locale, de manière systémique ou ex vivo.

12. Procédé pour la préparation d'une CPA telle que définie dans les revendications précédentes, comprenant la transduction ex vivo de la CPA avec un antigène tumoral et un antigène étranger capable de provoquer une réaction immunitaire dans l'organisme d'un patient.

13. Procédé suivant la revendication 12, dans lequel la CPA est un lymphocyte T.

14. Cellule présentatrice d'antigène transduite avec un antigène tumoral et un antigène étranger capable de provoquer une réaction immunitaire dans l'organisme d'un patient.

15. Cellule suivant la revendication 14, ladite cellule étant un lymphocyte T.

16. Cellule suivant la revendication 14 ou 15, ladite cellule étant transduite avec un gène de thymidine-kinase viral.

17. Cellule suivant la revendication 16, dans laquelle le gène de thymidine viral est un gène HSV-Tk.

18. Cellule suivant l'une quelconque des revendications 14 à 17, ladite cellule étant transduite avec un gène de résistance bactérien.

19. Cellule suivant la revendication 18, dans laquelle le gène bactérien code pour la résistance à la néomycine.

20. Cellule suivant l'une quelconque des revendications 14 à 19, dans laquelle l'antigène tumoral est choisi entre MAGE, BAGE, GAGE, un épitope de reconnaissance de CTL provenant de la tyrosinase, MelanA^{Mart1}, gp100^{me17}, gp75^{TRP1} et TRP-2.
